# EUROPEAN PATENT SPECIFICATION

(11) **EP 3 125 865 B1**
(45) Date of publication and mention of the grant of the patent: **22.05.2019**
(21) Application number: 15718629.7
(22) Date of filing: 01.04.2015
(51) Int. Cl.: A61K 31/164, A61K 31/575, A61K 31/20, A61K 9/00, A61K 47/12, A61K 47/18, A61K 47/20, A61K 47/28, A61K 9/107, A61K 47/26, A61P 17/16, A61K 8/36, A61K 8/63, A61K 8/68, A61Q 19/08, A61Q 19/10

(54) **ASSOCIATION OF ACTIVE INGREDIENTS FOR TOPICAL USE IN RESTRUCTURING ALTERED CUTANEOUS BARRIER FOLLOWING TO CUTANEOUS DISEASES**
ASSOZIATION VON WIRKSTOFFEN ZUR TOPISCHEN ANWENDUNG BEI DER RESTRUKTURIERUNG EINER VERÄNDERTEN HAUTBARRIERE NACH EINER HAUTKRANKHEIT
ASSOCIATION DE PRINCIPES ACTIFS ADMINISTRÉS PAR VOIE TOPIQUE POUR LA RESTRUCTURATION DE LA BARRIÈRE CUTANÉE MODIFIÉE SUITE À UNE MALADIE DE LA PEAU

(30) Priority: 04.04.2014 IT MI20140623
(43) Date of publication of application: 08.02.2017
(73) Proprietor: Unifarco S.p.A., 32035 Santa Giustina (BL) (IT)
(72) Inventor: BARATTO, Giovanni, I-32035 Santa Giustina (BL) (IT)
(74) Representative: Perani & Partners S.p.A.
(86) International application number: PCT/IB2015/052389
(87) International publication number: WO 2015/151040

(56) References cited:
- WO-A1-00/45786
- WO-A1-94/00127
- IT-B- 1 363 475
- AMATO L ET AL: "Corneometers researches confirm the efficacy of a new product containing ceramide 3, cholesterol and fatty acids in the treatment of secondary cutaneous xerosis. Results of a pilot study", MINERVA DERMATOLOGICA, EDIZIONI MINERVA MEDICA, TURIN, IT, vol. 137, no. 4, 1 January 2002 (2002-01-01), pages 281-286, XP009145005, ISSN: 0026-4741
- MAN MAO-QIANG ET AL: "Optimization of Physiological Lipid Mixtures for Barrier Repair.", JOURNAL OF INVESTIGATIVE DERMATOLOGY, vol. 106, no. 5, 1 May 1996 (1996-05-01), pages 1096-1101, XP055031349, ISSN: 0022-202X, DOI: 10.1111/1523-1747.ep12340135

## Description

### FIELD OF THE INVENTION

The present invention relates to an association of active ingredients for topic use in restructuring the altered cutaneous barrier following skin diseases such as psoriasis.

### BACKGROUND ART

Current topic emollient/moisturizing or protective formulations which are commercially available, when applied on skin altered due to skin diseases, carry out in most cases their emollient activity, creating a relatively impervious layer on the skin surface on which they are applied, having an effect more or less occlusive comparable to the effect of a plastic film applied on the skin surface itself to be protected and treated. Further, this layer which is hardly absorbable by the skin, is easily removable through the use of common detergents as well as by inadvertent mechanical action.

An improvement to such formulations is represented by the topic formulations described in the patent IT 1363475 to the Applicant.

The topic formulations described in said patent, which are characterized in that they contain ceramide, cholesterol, fatty acids of unknown chain length and for which it is not specified whether they are saturated fatty acids or whether they contain one ore more unsaturations, penetrate more deeply and thus can supplement the lipid amount of the epidermis whose barrier has been damaged. However such formulations, while been able to penetrate more deeply the skin layers, cannot act on the extracellular matrix and particularly on some types of proteins, such as for example filaggrin. Filaggrin belongs to the so-called S100 fused-type protein class. Profilaggrin, tricohyaline, hornerin, tricohyaline-like- 1, repetin, cornulin e filaggrin-2 belong to this class. Filaggrin plays a key role in the corneification process. Filaggrin is produced by the post-translational proteolysis of the precursor profilaggrin, promoting the aggregation of keratin filaments. Filaggrin 2, recently characterized, has been detected in the granular layer as well as in the corneal layer of the normally stratified epithelium, which is the common filaggrin distribution pathway. It has recently been observed that filaggrin- 2, as well as filaggrin, is low in patients suffering from atopic dermatitis, psoriasis vulgaris and ichthyosis vulgaris (T. Makino et al "Expression of filaggrin-2 protein in the epidermis of human skin diseases: A comparative analysis with filaggrin" Biochem. and Biophys. Research Communications 449(2014) 100-106. These findings are confirmed by the fact that the expression of podoplanin, a membrane-anchored and lymphangiogenesis-, platelet aggregation- and cell motility/invasion-associated glycoprotein, is inversely proportional to the formation of filaggrin in the psoriasic epidermis granular layer. (M. Honma et al "Podoplanin expression is inversely correlated with granular layer filaggrin formation in Psoriatic epidermis" Letters to Editor; Japanese Dermatological Association pages 296-297).

WO94/00127 describes a method for treating the epidermide of a terrestrial mammal suffering from a condition characterized by a perturbed epidermal barrier function, comprising topically administering a therapeutically effective amount of a composition comprising the lipids:
(a) cholesterol,
(b) an acyl ceramide and
(c) at least one fatty acid of from 12 to 20 carbon atoms in length in specific molar ratio among each other.

In this reference , not only experimental evidence is absent disclosing the alleged efficacy of the composition therein disclosed in the treatment of psoriasis, but it even results that the composition therein disclosed containing as the sole active ingredient the components (a), (b) and (c) is unable to restructure skin.

WO00/45786) discloses that cholesterol sulfate, when applied topically, is effective in that it enhances the cohesion of the stratum corneum resulting in a more prolonged retention of the stratum corneum.

### SUMMARY OF THE INVENTION

The Applicant has now discovered that an association comprising:
a) at least one ceramide,
b) at least one saturated fatty acid C₁₂-C₂₂,
c) cholesterol,
d) cholesterol sulfate,
can penetrate more deeply and more effectively in the skin, not only restoring the lipid supply of the altered skin, but also acting on the extracellular matrix by means of proteins on barrier filaggrin, which activity is completely lacking in the association described in IT1363475.

The effectiveness of the above-said association causes the relative topical compositions which contain it, to be used for treating psoriasis.

### DETAILED DESCRIPTION OF THE INVENTION.

As is known, ceramides are amides of saturated monocarboxylic acids with sphingosine, characterized by the following formula: 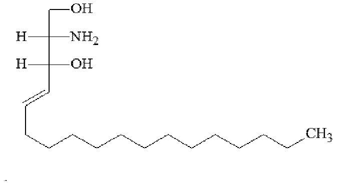 or with phytosphingosine i.e. the corresponding saturated form.

According to a preferred form of the association according to the present invention, ceramide is preferably selected from ceramide 2 i.e. N-acetylsphingosine (Ceramide NS), or ceramide 3 i.e. phytosphingosine amide (Ceramide NP) with stearic acid.

According to a particularly preferred form, the ceramide employed in the association according to the present invention is ceramide 3.

Preferably the saturated fatty acid or component b) of the association according to the present invention is a C₁₆-C₁₈ acid and, according to a particularly preferred embodiment, is stearic acid. Cholesterol sulfate or component d) of the association according to the present invention is preferably in salt form and even more preferably is in sodium salt form characterized by the following formula

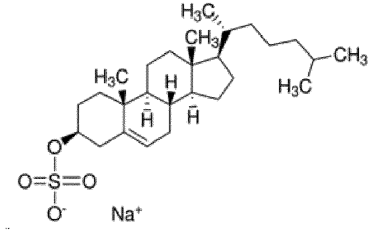

The topical compositions with which the association for use according to the present invention is formulated are preferably in the form of oil-in-water emulsions or water-in-oil emulsions.

Because of the particular association, subject of the present invention, oil-in-water formulations can be prepared which are substantially hydrophilic, such as for example hydrogels, or water-in-oil emulsions which are substantially lipophilic such as for example ointments, oils etc.

For the purposes of the present invention, with "substantially hydrophilic emulsion" is meant the oil-in-water emulsion wherein the aqueous phase is exclusive or prevailing, while with the term "substantially lipophilic emulsion" is meant the water-in-oil emulsion, wherein the oil phase is prevailing.

The topical compositions in the form of water-in-oil and oil-in-water emulsions according to the present invention preferably contain excipients, for example emulsifiers, emollient phase stabilizers, viscosifying polymers and rheological modifiers, variable polarity oils, natural or hydrocarbon waxes, varying chain silicone derivatives and polymers thereof commonly employed for preparing such emulsions and as described in Remington The Science and Practice of Pharmacy 21° Edition. The water-in-oil and oil-in-water emulsions according to the present invention are preferably in form of creams, detergent lotions, salves, lipogels or hydrogels, ointments, milks.

Preferably the association for use according to the present invention is formulated in compositions containing each of the components (a)-(d) in weight concentrations, calculated based on the total weight of the topical compositions, between 0.1 and 5%.

The association for use according to the present invention can be employed as a single therapy or as a therapy complementary to therapies for systemic administration and can be administered once or more than once daily depending on the severity of the disease.

Particularly preferred topical compositions containing the association for use according to the present invention are shown hereinafter. The active ingredients being part of the association subject of the invention are indicated in bold.

**1-Water-in-oil emulsion:**

| | |
|---|---|
| Water | 58.714 ÷ 67.011 |
| Liquid paraffin | 6.440 ÷ 7.350 |
| Hydrogenated polydecene | 1 ÷ 8 |
| Isohexadecane | 1÷ 10 |
| Pentylene glycol | 4.600 ÷ 5.250 |
| Petrolatum | 4.600 ÷ 5.250 |
| Squalane | 0.1 ÷ 5 |
| Butyrospermum parkii | 3.680 ÷ 4.200 |
| Glycerin | 3.662 ÷ 4.179 |
| Polyglyceryl-3 polyricinoleate | 1.840 ÷ 2.100 |
| Sorbitan isostearate | 1.840 ÷ 2.100 |
| Magnesium sulfate | 0.644 ÷ 0.735 |
| **Ceramide 3/2** | **0.1 ÷ 5** |
| Magnesium stearate | 0.460 ÷ 0.525 |
| **Cholesterol** | **0.1 ÷ 5** |
| **Cholesterol sulfate** | **0.1 ÷ 5** |
| **Stearic acid** | **0.1 ÷ 5** |

**2-Oil-in-water emulsion**

| | |
|---|---|
| Water | 56.548 ÷ 64.538 |
| Urea | 18.400 ÷ 21000 |
| Hydrogenated polydecene | 7.360 ÷ 8.400 |
| Butyrospermum parkii | 3.680 ÷ 4.200 |
| Glycerin | 2.746 ÷ 3,134 |
| Ammonium acryloyldimethyltaurate/VP (vinyl pyrrolidone) copolymer | 0,920 ÷ 1,050 |
| Palmitamide MEA (monoethylamine) | 0,920 ÷ 1,050 |
| Caprylyl glycol | 0,460 ÷ 0,525 |
| Hydroxyethyl acrylate/Sodium acryloyldimethyl taurate copolymer | 0,368 ÷ 0,420 |
| Xanthan gum | 0,184 ÷ 0,210 |
| o-Cimen-5-ol | 0,092 ÷ 0,105 |
| Polysorbate 60 | 0,092 ÷ 0,105 |
| Squalane | 0,1 ÷ 5 |
| **Ceramide 3** | **0,1 ÷ 5** |
| **Cholesterol** | **0,1 ÷ 5** |
| **Cholesterol sulfate** | **0,1 ÷ 5** |
| **Stearic acid** | **0,1 ÷ 5** |

**3. Face cleanser (hydrogel formulation)**

| | |
|---|---|
| Water | 79,525 ÷ 90,762 |
| Glycerin | 4,577 ÷ 5,224 |
| Dimethicone | 2,300 ÷ 2,625 |
| Dimethicone cross-polymer | 1,380 ÷ 1,575 |
| Tocopheryl acetate | 0,920 ÷ 1,050 |
| Ammonium acryloyldimethyltaurate/VP pyrrolidone) copolymer (vinyl | 0,736 ÷ 0,840 |
| Allantoin | 0,460 ÷ 0,525 |
| Caprylyl glycol | 0,460 ÷ 0,525 |
| Sodium acrylate/sodium acryloyldimethyl copolymer taurate | 0,368 ÷ 0,420 |
| Sodium hyaluronate | 0,368 ÷ 0,420 |
| Isohesadecane | 0,230 ÷ 0,263 |
| Dehydroxanthan gum | 0,184 ÷ 0,210 |
| Capryloyl glycine | 0,138 ÷ 0,158 |
| Lauryl glucoside | 0,124 ÷ 0,142 |
| Arginine | 0,110 ÷ 0,126 |
| Polysorbate 80 | 0,092 ÷ 0,105 |
| Squalane | 0,1 ÷ 5 |
| **Ceramide 3** | **0,1 ÷ 5** |
| **Cholesterol** | **0,1 ÷ 5** |
| **Cholesterol sulfate** | **0,1 ÷ 5** |
| **Stearic acid** | **0,1 ÷ 5** |

## Claims

1. An association comprising:
a) at least one ceramide,
b) at least one saturated fatty acid C₁₂-C₂₂,
c) cholesterol
d) cholesterol sulfate
for topic use in restructuring the altered cutaneous barrier following psoriasis.

2. The association for the use according to claim 1, wherein said association is in form of a topical composition, comprising said association as an active ingredient in combination with suitable excipients and/or diluents.

3. The association for the use according to claim 2, wherein said topical composition contains each component a), b), c) and d) in concentrations comprised between 0,1 and 5% by weight on the total weight of said composition.

4. The association for the use according to claim 2 or 3, wherein said topical composition is in form of oil in water emulsions.

5. The association for the use according to claim 4, wherein said oil in water emulsion is in form of substantially hydrophilic emulsions.

6. The association for the use according to claim 2 or 3, wherein said topical composition is in form of water in oil emulsions.

7. The association for the use according to claim 6, wherein said water in oil emulsion is in form of substantially lipophilic emulsions.

## Patentansprüche

1. Eine Verbindung bestehend aus:
a) mindestens einem Ceramid,
b) mindestens einer gesättigten Fettsäure C₁₂ - C₂₂,
c) Cholesterin,
d) Cholesterinsulfat
zur topischen Anwendung bei der Umstrukturierung der veränderten Hautbarriere nach Psoriasis.

2. Verbindung zur Verwendung nach Anspruch 1, wobei die Verbindung in Form einer topischen Zusammensetzung vorliegt, die die Verbindung als Wirkstoff in Kombination mit geeigneten Hilfsstoffen und/oder Verdünnungsmitteln umfasst.

3. Verbindung zur Verwendung nach Anspruch 2, wobei die topische Zusammensetzung jede Komponente a), b), c) und d) in Konzentrationen zwischen 0,1 und 5 Gew.-% des Gesamtgewichts der Zusammensetzung enthält.

4. Verbindung zur Verwendung nach Anspruch 2 oder 3, wobei die topische Zusammensetzung in Form von Öl-in-Wasser-Emulsionen vorliegt.

5. Verbindung zur Verwendung nach Anspruch 4, wobei die Öl-in-Wasser-Emulsion in Form von im Wesentlichen hydrophilen Emulsionen vorliegt.

6. Verbindung zur Verwendung nach Anspruch 2 oder 3, wobei die topische Zusammensetzung in Form von Wasser-in-Öl-Emulsionen vorliegt.

7. Verbindung zur Verwendung nach Anspruch 6, wobei die Wasser-in-Öl-Emulsion in Form von im Wesentlichen lipophilen Emulsionen vorliegt.

## Revendications

1. Une association comprenant :
a) au moins une céramide,
b) au moins un acide gras saturé en C₁₂ à C₂₂,
c) cholestérol,
d) sulfate de cholestérol
à utiliser par voie locale dans la restructuration de la barrière cutanée altérée à la suite du psoriasis.

2. Association pour l'utilisation selon la revendication 1, dans laquelle ladite association est sous la forme d'une composition topique, comprenant ladite association en tant qu'ingrédient actif en combinaison avec des excipients et/ou des diluants appropriés.

3. Association pour l'utilisation selon la revendication 2, dans laquelle ladite composition topique contient chaque composant a), b), c) et d) à des concentrations comprises entre 0,1 et 5% en poids du poids total de ladite composition.

4. Association pour l'utilisation selon la revendication 2 ou 3, dans laquelle ladite composition topique est sous forme d'émulsions huile dans eau.

5. Association pour l'utilisation selon la revendication 4, dans laquelle ladite émulsion huile dans eau est sous forme d'émulsions substantiellement hydrophiles.

6. Association pour l'utilisation selon la revendication 2 ou 3, dans laquelle ladite composition topique est sous forme d'émulsions eau dans huile.

7. Association pour l'utilisation selon la revendication 6, dans laquelle ladite émulsion eau dans huile est sous forme d'émulsions substantiellement lipophiles.
